# EUROPEAN PATENT SPECIFICATION

(11) **EP 3 840 587 B1**
(45) Date of publication and mention of the grant of the patent: **08.01.2025**
(21) Application number: 19756322.4
(22) Date of filing: 22.08.2019
(51) Int. Cl.: A23K 20/158, A23K 20/20, A23K 50/20, A23K 50/30

(54) **ANIMAL FEED FOR SUCKING PIGS**
TIERFUTTER FÜR SPANFERKEL
ALIMENT POUR ANIMAUX POUR COCHONS DE LAIT

(30) Priority: 23.08.2018 BE 201800092
(43) Date of publication of application: 30.06.2021
(73) Proprietor: Proviron Holding N.V., 2620 Hemiksem (BE)
(72) Inventor: VANHEULE, José, 2620 Hemiksem (BE); ROGGE, Tina, 2620 Hemiksem (BE); LAUWAERTS, Angelo, 2620 Hemiksem (BE)
(74) Representative: Theunis, Patrick
(86) International application number: PCT/EP2019/025281
(87) International publication number: WO 2020/038610

(56) References cited:
- EP-A1- 1 224 870
- CN-A- 105 410 365
- DATABASE MEDLINE [online] US NATIONAL LIBRARY OF MEDICINE (NLM), BETHESDA, MD, US; December 2012 (2012-12-01), MORALES J ET AL: "Zinc oxide at low supplementation level improves productive performance and health status of piglets.", Database accession no. NLM23365402
- MORALES J ET AL: "Zinc oxide at low supplementation level improves productive performance and health status of piglets.", JOURNAL OF ANIMAL SCIENCE DEC 2012, vol. 90 Suppl 4, December 2012 (2012-12-01), pages 436 - 438, ISSN: 1525-3163
- YUAN S: "Pig feed used for promoting digestion and preventing diarrhea, includes corn, extruded soybean, fermented peanut meal, protein whey powder, plasma protein powder, mulberry leaf powder, pupa powder, earthworm powder, phytase, and plant oil", WPI / 2017 CLARIVATE ANALYTICS,, vol. 2016, no. 42, 24 February 2016 (2016-02-24), XP002769783
- DATABASE BIOSIS [online] BIOSCIENCES INFORMATION SERVICE, PHILADELPHIA, PA, US; December 2012 (2012-12-01), MORALES J ET AL: "Zinc oxide at low supplementation level improves productive performance and health status of piglets", XP002787302, Database accession no. PREV201300485180
- JOURNAL OF ANIMAL SCIENCE, vol. 90, no. Suppl. 4, December 2012 (2012-12-01), pages 436 - 438, ISSN: 0021-8812(print), DOI: 10.2527/JAS.53833
- OU ET AL: "Dietary supplementation with zinc oxide decreases expression of the stem cell factor in the small intestine of weanling pigs", THE JOURNAL OF NUTRITIONAL BIOCHEMISTRY, ELSEVIER, AMSTERDAM, NL, vol. 18, no. 12, 23 November 2007 (2007-11-23), pages 820 - 826, XP022360168, ISSN: 0955-2863, DOI: 10.1016/J.JNUTBIO.2006.12.022
- PIVA A ET AL: "Intestinal metabolism of weaned piglets fed a typical United States or European diet with or without supplementation of tributyrin and lactitol", JOURNAL OF ANIMAL SCIENCE, AMERICAN SOCIETY OF ANIMAL SCIENCE, US, vol. 86, no. 11, 31 October 2008 (2008-10-31), pages 2952 - 2961, XP009509894, ISSN: 0021-8812, DOI: 10.2527/JAS.2007-0402

## Description

### Field of the invention

The present invention relates to feed composition for sucking pigs.

More in particular, the present invention relates to a feed composition for sucking pigs, comprising glycerol tri butyrate and monolaurate. The function of these additives is to enhance the immunity for diseases of the treated animals.

The inventors have found that a feed comprising such composition yields a surprisingly beneficial effect on the growth and health during the breeding of sucking pigs.

### Background of the invention

### Problem to be solved

It is known that the breeding and growth of sucking pigs can be seriously hampered by the outburst of diseases. A particular case of such diseases is the presence of bacterial infections.

This kind of diseases occur inter alia at the occasion of the transition from milk feeding to adult feeding of sucking pigs. At this stage stomach and intestines related problems, such as diarrhea, often occur.

A known method to solve these problems comprises feeding antibiotics to the young animals. However, given the increasing tendency to ban antibiotics from animal feed, the feeding of high amounts of zinc oxide has been commonly applied as a legally acceptable alternative.

By the addition of zinc oxide two goals are fulfilled at the same time: the absorption of zinc as a necessary mineral for the body of the sucking pigs and the antimicrobial effect of zinc oxide. This product usually has been added in an amount of a few kg per ton of animal feed.

Meanwhile the addition of 3 up to 4 kg of zinc oxide to the animal feed in particular for sucking pigs, has become common practice as a replacement for antibiotics.

Antibiotics used to be fed in the form of various 'cocktails', or the composition was changed depending on time and age of the sucking pigs.

The diseases as mentioned supra can be treated in a satisfactorily manner in this way.

However, the large-scale use of zinc oxide gives rise to problems, notably problems from the point of view of ecology.

The animal to be fed, such as the sucking pig, has a need for about 100 up to 150 gr of Zinc oxide, the remaining part of the zinc oxide fed not being absorbed by the animal, and being excreted. The limited amount of 100 up to 150 gr usually is sufficient as mineral for the body of the sucking pigs. The remaining part is used to kill the E. colli-bacteria, that disrupt the balance in the intestines, or at least to keep their presence within healthy limits. This part of the zinc is not absorbed by the sucking pig and is excreted by the animal.

The so excreted, excess zinc finally ends up in the farm manure, and so finally in the soil that is enriched with this heavy metal; from an ecological point of view, this is unacceptable.

The use of zinc up to an amount of 150 ppm still is allowed, but the addition of this amount to animal feed is not high enough to solve the problems set forth above. The article published in WPI/2017 Clarivate Analytics, part 2016, Nr. 42 on February 24, 2016, by Yuan S (Pig feed used for promoting digestion and preventing diarrhea, includes corn, ...) (XP002769783) discloses the joint use of zinc oxide and glycerol tri butyrate in animal feed compositions.

Although the use of such a combination may be interesting from a scientific point of view; from a business point of view however, irrespective of the healthy effect caused by such a feed composition, the cost of the feed is way too high for such composition to be used under realistic economic conditions.

Therefore, the need for a healthy feed supplement, corresponding to an economically realistic cost, and not causing concerns from an ecological point of view, remains.

The Chinese patent application published as CN 105 410 365 A, by the University of Zhejiang, HZKY Food Science and Tech Co. Ltd, published march 23, 2016, discloses an animal feed comprising a mixture of glycerol monolaurate in an amount of 10 up to 20 %, glycerol di-laurate in an amount of 20 up to 40%, and glycerol monostearate in an amount of 30 up to 60 %. This composition is aimed at giving the animal feed a pleasant taste. Further the presence of glycerol monostearate is required for emulsification and synergetic effect.

Further it is stressed in this application that "the dosage of glycerol monolaurate, glycerol di laurate and glycerol mono stearate in the feed additive of the invention must be suitable, otherwise the feed additive will be too strong due to mouthfeel and antibacterial and antiviral effects..."

The inventors have devised and embodied the present invention to overcome these shortcomings and to obtain further advantages.

### Summary of the invention

The goal of the present inventors is the search and development of functional compositions that are able to adequately solve the above cited problems when added as additive or supplement to animal feeds, in particular for sucking pigs. More in particular, the aim of the inventors is the development of feed additives that have a beneficial effect on the metabolism of sucking pigs so as to prevent or minimize the occurrence of bacterial and other infections with sucking pigs. Finally, the aim of the inventors is also to develop a feed additive that does not give rise to the ecological issues as abovementioned.

These objects and advantages are given only by way of illustrative example, and such objects may be exemplary of one or more embodiments of the invention. Other desirable objectives and advantages inherently achieved by the disclosed invention may occur or become apparent to those skilled in the art.

### Statement of invention:

The present inventors have conducted extensive studies in order to solve the above-mentioned problems. As a result, they have successfully found that the aims and goals set forth above are successfully reached by the use of an animal feed as set forth in the appended claims.

The invention is defined and characterized in the main claim, while the dependent claims describe other characteristics and specific features for preferred embodiments of the invention.

Further aspects and advantages of the embodiments described will appear from the following description.

### Detailed description of embodiments of the invention

The following is a detailed description of the preferred embodiments of the invention.

The inventors have performed various experiments and tests, whereby various components in varying amounts have been added to animal feeds. These tests in particular relate to animal feed that has been fed to sucking pigs.

The term 'sucking pigs' in the context of the present invention should be understood to relate to young pigs in or shortly after the transition from milk feeding to firm or non-liquid feeding.

From the experiments and tests described hereinafter, it has appeared that compositions comprising glycerol tri butyrate, glycerol mono laurate and zinc oxide are particularly effective and so can be added to animal feed for pigs, sucking pigs in particular.

Such a composition can also be added as a feed supplement for sucking pigs. More in particular, from the experiments as described hereinafter, it has appeared that when sucking pigs are fed with an animal feed comprising such a supplement or composition, the health in general of the animals so fed improves in a similar manner as when these sucking pigs are fed with a feed comprising larger amounts of zinc oxide.

Notably, when the animal feeds according to the present invention are used, the problems of an ecological nature as described above, do not occur.

It appears that such a supplement or composition, added to a standard animal feed for sucking pigs, substantially enhances the immunity of these animals. More in particular, the immunity of the animals is increased in a similar manner as compared to the situation whereby the animals are fed on the basis of a feed comprising high amounts of zinc oxide as a supplement.

The advantage of the use of the supplement according to the invention is that the environment is not encumbered with the heavy metal zinc.

So as to obtain such effect, it is sufficient that the supplement or composition is added to an animal feed according to the invention in an amount comprised between 4 up to 6, preferably about 5 kg per ton of animal feed. Higher amounts are not required; they do not yield any additional beneficial effect.

The supplement comprises between 50 and 200, more preferably between 100 and 150 g of zinc oxide.

According to a preferred embodiment of the invention, the relative weight amount of glycerol tri butyrate with respect to glycerol mono laurate is comprised within 40/60 up to 60/40 %.

### Glycerol tri butyrate:

Glycerol tri butyrate is known as a product as such and can be obtained for example by the esterification of glycerol with the corresponding organic carboxylic acid, butyric acid.

This synthesis step may be followed by a purification step in order to remove the excess of raw materials that were used.

The esterification of the alcohol, in this case glycerol, with the organic acid takes place according to the reaction mechanism set forth hereinbelow:

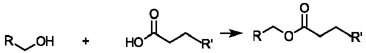

The structural formula of butyric acid is as follows:

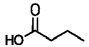

Butyric acid is a carboxylic acid comprising four carbon atoms.

The structural chemical formula of glycerol tri butyrate is as follows:

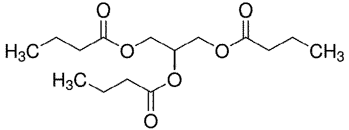

Glycerol tri butyrate is a low viscous product, insoluble in water, with a melting point below 40°C. It is available in liquid state or in solid state. In the latter case, it is often available as a 70 % liquid placed on an inert carrier, in most cases silica. In a preferred embodiment of the present invention, glycerol tri butyrate is comprised as a solid product, placed on an inert carrier, for example silica, in the feed supplement of the present invention and as such added to the animal feed.

In the feed supplement according to the invention, as well as in the animal feed according to the invention, the glycerol tri butyrate is used in combination with glycerol mono laurate.

When the glycerol tri butyrate is deposited solely on an inert carrier such as silica, the silica accounts for approximately 10 % of the total weight. When apart from glycerol tri butyrate also glycerol mono laurate has been deposited, the silica accounts for approximately 30 % of the total weight.

### Glycerol mono laurate.

Glycerol mono laurate is the esterification product of glycerol with lauric acid. The latter is a carboxylic acid comprising twelve carbon atoms.

The structure of this compound is shown hereinafter:

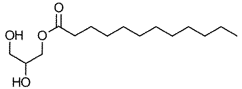

The synthesis of glycerol mono laurate follows a similar process as said forth above for glycerol tri butyrate, using lauric acid as starting material instead of butyric acid.

According to a preferred embodiment, the necessary steps and precautions are taken during the synthesis of the glycerol mono laurate for use in the supplement according to the present invention, that mainly, preferably at least 90 %, of monolaurate is obtained as a result of the synthesis and purification by distillation, the amount of by-products such as glycerol di-laurate being 10 % or less.

As will be apparent from the tests described hereinafter, surprisingly good results have been noted when highly reduced amounts of zinc oxide have been used in combination with limited amounts of glycerol tri butyrate and minor quantities of purified glycerol monolaurate, preferably without use of any additional emulsifier, in animal feed as a means for enhancing the immunity of sucking pigs.

### Tests performed

During the tests, sucking pigs have been fed with a traditional, state-of-the-art animal feed, to which on the one hand a control supplement and on the other hand the feed supplement according to the invention have been added.

A typical composition for a state-of-the-art animal feed is for example as follows: (the figures between brackets show the weight percentage of the respective compound in the total animal feed composition)

| | |
|---|---|
| Corn gluten | 5,00 % |
| Danish fish flour | 26,00 % |
| Squid fish flour | 2,00% |
| Wheat | 16,90 % |
| Wheat flour | 25,00% |
| Soybean flour | 15,00% |
| Soy lecithin | 2,00% |
| Fish oil | 2,00% |
| Wheat gluten | 4,00% |
| Premix | 2,00% |
| Cholesterol | 0,10% |
| Total | 100% |

The premix is a mixture of vitamins and minerals.

Soy lecithin acts as an emulsifier.

To this animal feed, the feed supplement according to the invention on the one hand, and according to the state of the art on the other hand (pure zinc oxide, control test) have been added.

The animal feed supplements that have been tested, are as follows:
- on the one hand, the control feed supplement, namely zinc oxide, added in an amount of 3 kg of Zinc oxide per ton of animal feed (= the control animal feed);
- on the other hand, the feed supplement according to the invention, namely 150 g of zinc oxide and 5 kg of glycerol tri butyrate and glycerol monolaurate (in a mutual weight ratio of 60/40% (= the feed supplement or additive composition, resp. the animal feed according to the invention), deposited on silica as inert carrier.

The glycerides can be added in amounts varying from 2, 3, 4, 5, 6, 7 up to 8 kg per ton of animal feed.

Each of these two animal feeds, namely the control animal feed and the animal feed according to the invention, have been added to approximately eighty sucking pigs, distributed over 8 cages of each approximately 10 pigs per cage.

The sucking pigs were 28 days old.

The tests ran over a period of four weeks after weaning, the animals being fed with the animal feeds as set forth above. Apart from the so-called reference or control composition (on the basis of 3 kg of zinc oxide per ton of feed) animals were also fed with an animal feed to which 150 g of zinc oxide per ton of feed and 5 kg of glycerol tri butyrate and glycerol monolaurate in a mutual weight ratio of 60/40% had been added.

The weight of the sucking pigs was measured at the start of the test (at the start of the weaning) and 14, resp. 28 days after weaning.

The consumption of animal feed was measured on a weekly basis per cage.

The increase in weight was measured for the first 2 and 4 weeks after weaning and for the total duration of the test.

The Feed Conversion Rate (FCR) was determined for the first 2 and 4 weeks after weaning, and for the total duration of the test.

Specimen of the faeces were investigated for microbiological analysis upon weaning and at day 14 and 28 after weaning.

At the end of the test period, eight pigs for each test (control and according to the invention) were subject of an histological evaluation of the health condition of the intestines. A number of issues were checked in this respect.

The surprising result of these tests is that no noticeable effect has been determined with respect to the growth or the weight increase of the animals as a function of the various feeds that were fed, namely the one according to the control and the one according to the invention.

Differently phrased, the growth and the increase in weight of the animals was not significantly different whether they were fed with the reference diet or the control diet, or with the diet comprising the composition according to the invention.

As an illustration of the above data, the results as obtained in these tests are shown in the following tables:

**Table 1**

| Treatment effect on the weight and the coefficient of variation of the sucking pigs: | | |
|---|---|---|
| **Weight, in kg** | **Control** | **Invention** |
| At the start | 6.75 | 6.80 |
| After 14 days | 9.64 | 9.75 |
| After 28 days | 14.73 | 14.65 |

| **Coefficient of variation, in %** | | |
|---|---|---|
| At the start | 19.19 | 18.55 |
| After 14 days | 22.14 | 17.49 |
| After 28 days | 22.31 | 19.24 |

**Table 2**

| Effect of the treatment, average daily increase in weight | | |
|---|---|---|
| Weight, **in kg** | **Control** | **Invention** |
| 0-14 days | 0.199 | 0.201 |
| 14-28 days | 0.363 | 0.364 |
| 0-28 days | 0.281 | 0.283 |

The figures mentioned above show the average daily increase in weight of the sucking pigs (ADG, Average Daily Gain), for the periods as mentioned, this is 0-14, resp. 14-28 and 0-28 days after weaning.

As is apparent from this table, the difference in weight increase for the sucking pigs fed with the control or the reference feed and these fed with the feed according to the invention is statistically not significant.

From this, one may conclude that the effect of the feed according to the invention practically equals the effect of the feed according to the reference.

**Table 3**

| Treatment effect on sucking pigs, average daily feed intake | | |
|---|---|---|
| **ADFI, in kg** | **Control** | **Invention** |
| 0-7 days after weaning | 0.229 | 0.236 |
| 7-14 days after weaning | 0.445 | 0.385 |
| 14-21 days after weaning | 0.677 | 0.655 |
| 21-28 days after weaning | 0.791 | 0.761 |
| 0-28 days after weaning | 0.536 | 0.509 |

The figures as abovementioned show the average daily feed intake of the sucking pigs (ADFI, Average Daily Feed Intake), for the periods as mentioned, this is 0-7, resp. 7-14, 14-21, 21-28 and 0-28 days after the start of weaning.

As is apparent from this table, the difference in average daily feed intake for the sucking pigs fed with the control or reference feed and those fed with the feed according to the invention, is statistically not significant.

From this, one may conclude that the effect of the feed according to the invention practically equals the effect of the reference feed.

**Table 4:**

| Effect of the treatment, feed conversion ratio (FCR) | | |
|---|---|---|
| **FCR** | **Control** | **Invention** |
| 0-14 days after weaning | 1.72 | 1.56 |
| 14-28 days after weaning | 2.04 | 1.95 |
| 0-28 days after weaning | 1.88 | 1.76 |

The figures abovementioned show the feed conversion rate (FCR) for the periods as mentioned, this is 0-14, resp. 14-28 and 0-28 days after weaning. The term weaning should be understood as the practice whereby sucking pigs are made independent from suckling, or are separated from the breast of the sow. This happens suddenly, without transition. At the age of 3 to 5 weeks, the pigs are separated from the sow and kept in separate cages in groups of approximately ten pigs.

As is apparent from this table, the difference in numbers for sucking pigs fed with the control or reference feed and those fed with the feed according to the invention, is statistically not significant.

From this, one may conclude that the effect of the feed according to the invention practically equals the effect of the reference feed.

**Table 5.1**

| Occurrence of diarrhea with sucking pigs, per cage, control feed | | | | |
|---|---|---|---|---|
| **Date** | **1** | **2** | **3** | **4** |
| 27/3/2018 | 1/1 | 1/1 | 1/1 | 1/1 |
| 28/3/2018 | 3/3 | 2/2 | 1/1 | 1/1 |
| 29/3/2018 | 3/2 | 2/1 | 1/1 | 1/2 |
| 30/3/2018 | 1/1 | 1/1 | 1/1 | 1/2 |
| 31/3/2018 | 2/2 | 1/1 | 1/1 | 2/2 |
| 1/4/2018 | 1/1 | 1/2 | 1/1 | 2/2 |
| 2/4/2018 | 1/2 | 1/2 | 1/1 | 1/2 |
| 3/4/2018 | 1/1 | 1/1.5 | 1/1 | 1/1.5 |
| 4/4/2018 | 1.5/1 | 1.5/1 | 1/1 | 1.5/1 |
| 5/4/2018 | 1/1 | 1/1 | 1/1 | 1/1 |
| 6/4/2018 | 1/1 | 1/1 | 1/1 | 1/1 |

**Table 5.2**

| Occurrence of diarrhea with sucking pigs, per cage, fed according to the invention | | | | | |
|---|---|---|---|---|---|
| **Date** | **1** | **2** | **3** | **4** | |
| 27/3/2018 | | 1/1 | 1/1 | 1/1 | 1/1 |
| 28/3/2018 | | 2/2 | 2/2 | 2/2 | 1/1 |
| 29/3/2018 | | 3/4 | 3/4 | 3/4 | 2/4 |
| 30/3/2018 | | 3/3 | 2/2 | 2/3 | 4/4 |
| 31/3/2018 | | 3/3 | 3/3 | 3/3 | 3/3 |
| 1/4/2018 | | 2/2 | 2/2 | 2/2 | 2/2 |
| 2/4/2018 | | 2/2 | 2/1 | 1/2 | 1/1 |
| 3/4/2018 | | 1/1.5 | 1/1 | 1/1.5 | 1/1.5 |
| 4/4/2018 | | 1.5/1 | 1/1 | 1.5/1 | 1.5/1 |
| 5/4/2018 | | 1/1 | 1/1 | 1/1 | 1/1 |
| 6/4/2018 | | 1/1 | 1/1 | 1/1 | 1/1 |

The above tables show the occurrence of diarrhea with sucking pigs in the various cages. The first figure shows the result for the day indicated in the morning, the second figure shows the result of the day mentioned in the afternoon. These figures indicate the degree of diarrhea on a qualitative scale ranging from 0 (normal state) up to 4 (heavy diarrhea).

Table 5.1 shows the results with respect to the reference or control feed; table 5.2 shows the results with respect to the feed according to the invention.

As is apparent from these tables, the difference in results for the sucking pigs fed with the control or reference feed, and those fed with the feed according to the invention, is statistically not significant.

From these results, one may conclude that the effect of the feed according to the invention practically equals the effects of the reference feed.

The result of these experiments is that the control on the occurrence of diarrhea with the sucking pigs fed with the feed according to the invention, in comparison with the situation when a high dose of zinc oxide is used, is similar. Differently phrased, a high dose of zinc oxide does not control the diarrhea better than the use of the composition according to the invention. A statistically significant difference has not been noted with respect to the results between both treatments.

**Table 6.1**

| Microbiological analysis, sucking pigs fed with the control feed | | | | | |
|---|---|---|---|---|---|
| | ***E. coil*** | ***Streptococcus*** | ***Lactobacillus*** | ***Clostridium perfringens*** | ***Pseudomonas*** |
| **Ca.1** | 6,25×10⁶ | 4.45×10⁴ | 3.4×10⁴ | 8.4×10⁴ | 5.3×10⁷ |
| **Ca.2** | 1.45×10⁶ | 1×10³ | 6.4×10⁴ | 5.85×10⁴ | 7.75×10⁷ |
| **Ca.3** | 7.4×10⁶ | 4.1×10⁴ | 8.65×10⁴ | 6.05×10⁴ | 4.75×10⁷ |
| **Ca.4** | 8×10⁵ | 8.65×10⁴ | 4.3×10⁴ | 2×10⁴ | 6.4×10⁶ |
| **Cb.1** | 4.95×10⁶ | 5.5×10⁴ | 5.55×10⁴ | 5.8×10⁴ | 5.5×10⁶ |
| **Cb.2** | 7.2×10⁶ | 7.25×10⁴ | 1.31×10⁵ | 6.65×10⁴ | 4.25×10⁷ |
| **Cb.3** | 1.3×10⁶ | 0.5×10³ | 5.25×10⁴ | 5×10⁴ | 7.3×10⁷ |
| **Cb.4** | 7,05×10⁶ | 2.15×10⁴ | 3.85×10⁴ | 4.2×10⁴ | 5.05×10⁷ |
| **Cc.1** | 8.5×10⁵ | 2.7×10⁴ | 1.56×10⁵ | 5×10⁴ | 7×10⁶ |
| **Cc.2** | 5.25×10⁶ | 4.5×10⁴ | 5×10⁴ | 1.12×10⁵ | 5.6×10⁷ |
| **Cc.3** | 5.7×10⁶ | 7.4×10⁴ | 4.45×10⁴ | 6.15×10⁴ | 1.39×10⁸ |
| **Cc.4** | 2.25×10⁶ | 5×10³ | 7.3×10⁴ | 4.8×10⁴ | 5.8×10⁷ |

In the above table, the letter C represents the Control Feed, the letter a indicates the start of the weaning, the letter b indicates the time 14 days after the start of the weaning, the letter c indicates the time 28 days after the start of the weaning. The figures 1, 2, 3 and 4 indicate the cage wherein the sucking pigs reside.

The numbers indicated for these points in time and sucking pigs indicate the number of bacterial counts (cfu) resulting from the microbiological analyses for the indicated bacteria.

**Table 6.2**

| Microbiological analysis, sucking pigs fed with the feed according to the invention | | | | | |
|---|---|---|---|---|---|
| | ***E.coli*** | ***Streptococcus*** | ***Lactobacillus*** | ***Clostridium perfringens*** | ***Pseudomonas*** |
| **Pa.1** | 5.1×20⁶ | 6.35×10⁴ | 8.85×10⁴ | 2.75×10⁴ | 2.61×10⁸ |
| **Pa.2** | 7.35×10⁶ | 6.4×10⁴ | 6.45×10⁴ | 3.15×10⁴ | 5.85×10⁷ |
| **Pa.3** | 3.65×10⁶ | 3.37×10⁵ | 4.4×10⁴ | 7.75×10⁴ | 6.8×10⁷ |
| **Pa.4** | 8.55×10⁶ | 9.45×10⁴ | 5.35×10⁴ | 8.7×10⁴ | 3.58×10⁸ |
| **Pb.1** | 2.53×10⁷ | 4.35×10⁴ | 5.2×10⁶ | 3.75×10⁴ | 4.75×10⁷ |
| **Pb.2** | 9.6×10⁶ | 6.1×10⁴ | 8×10⁶ | 3.6×10⁴ | 5.2×10⁷ |
| **Pb.3** | 2.9×10⁶ | 1.71×10⁵ | 7.6×10⁶ | 3.4×10⁴ | 2.26×10⁸ |
| **Pb.4** | 6.05×10⁶ | 8.25×10⁴ | 3.25×10⁶ | 7.55×10⁴ | 5.4×10⁷ |
| **Pc.1** | 6.5×10⁵ | 5.6×10² | 4.6×10⁶ | 6.55×10⁴ | 2.66×10⁸ |
| **Pc.2** | 7.75×10⁶ | 1.13×10⁴ | 6.05×10⁶ | 8.85×10⁴ | 4.55×10⁷ |
| **Pc.3** | 6×10⁵ | 3.4×10² | 3.65×10⁶ | 2.2×10⁴ | 4.97×10⁸ |
| **Pc.4** | 1.05×10⁶ | 7.65×10² | 3.4×10⁶ | 4.1×10⁴ | 6.5×10⁶ |

In the above table, the number P indicates the feed according to the invention; the characters a, b and c and the numbers 1, 2, 3 and 4 have the same meaning as in table 6.1.

The numbers cited for these points in time have the same meaning as the corresponding numbers in table 6.1.

As is apparent from these tables, the difference in numbers for the sucking pigs fed with the control or the reference feed and those fed with the feed according to the invention, is statistically not relevant.

From this, one may conclude that the effect of the feed according to the invention is not different from the effect of the reference feed.

### General Conclusion

From the above tests is appears that by the addition of the composition to the feed for pigs, sucking pigs in particular, according to the invention, an enhancement of the immunity has been realized, resulting in a more effective protection of the animals against the occurrence of infections.

The weight amount of zinc oxide in the feed is comprised between 50 and 200 g per ton of feed, more preferably between 100 and 150 g per ton of feed.

The weight amount of glycerol tri butyrate and glycerol mono laurate together in the feed is comprised between 2 and 6 kg per ton of feed.

According to a still more preferred embodiment, this amount is situated between 4 and 6 kg per ton of feed, both products being deposited on an inert carrier, namely silica.

By the use of the feed composition according to the invention, the health of sucking pigs, more in particular with respect to the occurrence of or the control on the presence of diarrhea, is comparable to the situation whereby only pure zinc oxide, but in a markedly higher amount, is being used.

The big advantage of the feed composition according to the invention is that the problems arising from the accumulation of the secreted redundant zinc oxide in the environment in case the feed composition according to the invention is used, do not occur.

## Claims

1. Animal feed for pigs, comprising:
- glycerol tri butyrate and glycerol mono laurate, in a weight amount, of between 2 and 6 kg per ton of the animal feed, wherein the weight ratio of glycerol tri butyrate and glycerol mono laurate is comprised between 60% to 40 % and 40% to 60 %, and
- zinc oxide in a weight amount of between 50 and 200 gr per ton of the animal feed.

2. Animal feed according to claim 1, comprising the glycerol tri butyrate and glycerol mono laurate in a weight amount of between 4 up to 6 kg per ton of the animal feed.

3. Animal feed according to claim 1 or 2, comprising the zinc oxide in a weight amount of between 100 and 150 g per ton of the animal feed.

4. Animal feed according to claim 1, comprising the glycerol tri butyrate and glycerol mono laurate in a weight amount of 5 kg per ton of the animal feed in a mutual weight ratio of 60/40 %, and comprising zinc oxide in a weight amount of 150 g per ton of the animal feed,

5. Animal feed according to claim 1, wherein the glycerol tri butyrate and preferably also the glycerol mono laurate are deposited on an inert carrier.

6. Animal feed according to claim 5, wherein the inert carrier is silica.

7. Animal feed according to claim 5 or 6, wherein the weight amount of the inert carrier accounts for 10 up to 40 % of the weight of, more preferably accounts for 20 to 30 % of the weight of glycerol tri butyrate and glycerol mono laurate.

## Patentansprüche

1. Tierfutter für Schweine, enthaltend:
- Tributtersäureglycerinester und Monolaurin in einer Gewichtsmenge zwischen 2 und 6 kg pro Tonne des Tierfutters, wobei das Gewichtsverhältnis von Tributtersäureglycerinester und Monolaurin zwischen 60 % bis 40 % und 40 % bis 60 % liegt, und
- Zinkoxid in einer Gewichtsmenge zwischen 50 und 200 g pro Tonne des Tierfutters.

2. Tierfutter nach Anspruch 1, welches Tributtersäureglycerinester und Monolaurin in einer Gewichtsmenge zwischen 4 bis 6 kg pro Tonne des Tierfutters enthält.

3. Tierfutter nach Anspruch 1 oder 2, welches Zinkoxid in einer Gewichtsmenge zwischen 100 und 150 g pro Tonne des Tierfutters enthält.

4. Tierfutter nach Anspruch 1, enthaltend Tributtersäureglycerinester und Monolaurin in einer Gewichtsmenge von 5 kg pro Tonne des Tierfutters in einem gegenseitigen Gewichtsverhältnis von 60/40 %, und Zinkoxid in einer Gewichtsmenge von 150 g pro Tonne des Tierfutters.

5. Tierfutter nach Anspruch 1, wobei das Tributtersäureglycerinester und vorzugsweise auch das Monolaurin auf einem inerten Träger aufgebracht sind.

6. Tierfutter nach Anspruch 5, wobei der inerte Träger Siliciumdioxid ist.

7. Tierfutter nach Anspruch 5 oder 6, wobei die Gewichtsmenge des inerten Trägers 10 bis 40 % des Gewichts, vorzugsweise 20 bis 30 % des Gewichts, von Tributtersäureglycerinester und Monolaurin ausmacht.

## Revendications

1. Alimentation animale pour cochons, comprenant:
- glycérol tri butyrate et glycérol monolaurate, dans une quantité en poids, entre 2 et 6 kg par tonne de l'alimentation animale, dans laquelle la proportion en poids du glycérol tri butyrate et du glycérol monolaurate est comprise entre 60 % à 40 % et 40 % à 60 %, et
- l'oxide de zinc dans une quantité en poids entre 50 et 200 gr par tonne de l'alimentation animale.

2. Alimentation animale selon la revendication 1, comprenant le glycérol tri butyrate et le glycérol monolaurate dans une quantité en poids entre 4 à 6 kg par tonne de l'alimentation animale.

3. Alimentation animale selon la revendication 1 ou 2, comprenant l'oxide de zinc dans une quantité en poids entre 100 et 150 gr par tonne de l'alimentation animale.

4. Alimentation animale selon la revendication 1, comprenant le glycérol tri butyrate et le glycérol monolaurate dans une quantité en poids de 5 kg par tonne de l'alimentation animale dans une proportion mutuelle en poids de 60/40 %, et comprenant l'oxide de zinc dans une quantité en poids de 150 g par tonne de l'alimentation animale.

5. Alimentation animale selon la revendication 1, dans laquelle le glycérol tri butyrate et de préférence aussi le glycérol monolaurate sont déposés sur un porteur inerte.

6. Alimentation animale selon la revendication 5, dans laquelle le porteur inerte est le silica.

7. Alimentation animale selon la revendication 5 ou 6, dans laquelle la quantité en poids du porteur inerte représente entre 10 à 40 % du poids, de préférence représente entre 20 à 30 % de la quantité du glycérol tri butyrate et du glycérol monolaurate.
